(19) Europäisches Patentamt / European Patent Office / Office européen des brevets

(11) **EP 3 105 693 B1**

(12) **FASCICULE DE BREVET EUROPEEN**

(45) Date de publication et mention
de la délivrance du brevet:
**10.07.2019 Bulletin 2019/28**

(21) Numéro de dépôt: **15704776.2**

(22) Date de dépôt: **10.02.2015**

(51) Int Cl.:
**G06K 9/48** $^{(2006.01)}$  **G06K 9/62** $^{(2006.01)}$
**G06F 17/14** $^{(2006.01)}$  **G06K 9/00** $^{(2006.01)}$
**G06T 7/00** $^{(2017.01)}$

(86) Numéro de dépôt international:
**PCT/EP2015/052798**

(87) Numéro de publication internationale:
**WO 2015/121269 (20.08.2015 Gazette 2015/33)**

(54) **PROCEDE DE DETERMINATION D'UN MOTIF D'AUTO-ASSEMBLAGE D'UN COPOLYMERE A BLOCS**

VERFAHREN ZUM DEFINIEREN EINER SELBSTANORDNENDEN EINHEIT EINES BLOCKCOPOLYMERS

METHOD FOR DEFINING A SELF-ASSEMBLING UNIT OF A BLOCK COPOLYMER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.02.2014 FR 1451085**

(43) Date de publication de la demande:
**21.12.2016 Bulletin 2016/51**

(73) Titulaire: **Commissariat à l'Énergie Atomique et aux Énergies Alternatives 75015 Paris (FR)**

(72) Inventeur: **BELLEDENT, Jérôme F-38240 Meylan (FR)**

(74) Mandataire: **Priori, Enrico Marks & Clerk France Immeuble "Visium" 22, avenue Aristide Briand 94117 Arcueil Cedex (FR)**

(56) Documents cités:
- CHEN Q. ET AL: "Directed self-assembly of a colloidal kagome lattice", NATURE, vol. 469, no. 7330, 19 janvier 2011 (2011-01-19), pages 381-384, XP055156645, ISSN: 0028-0836, DOI: 10.1038/nature09713
- MÜLLER M. ET AL: "Computational Approaches for the Dynamics of Structure Formation in Self-Assembling Polymeric Materials", ANNUAL REVIEW OF MATERIALS RESEARCH, vol. 43, no. 1, 1 mai 2013 (2013-05-01), pages 1-34, XP055152976, ISSN: 1531-7331, DOI: 10.1146/annurev-matsci-071312-121618
- KIM H-CH. ET AL: "Block copolymer based nanostructures: materials, processes, and applications to electronics", CHEMICAL REVIEWS, vol. 110, no. 1, 1 décembre 2009 (2009-12-01), pages 146-177, XP055156903, ISSN: 0009-2665, DOI: 10.1021/cr900159v
- JEONG S-J. ET AL: "Directed self-assembly of block copolymers for next generation nanolithography", MATERIALS TODAY, vol. 16, no. 12, 1 décembre 2013 (2013-12-01), pages 468-476, XP055152922, ISSN: 1369-7021, DOI: 10.1016/j.mattod.2013.11.002

## Description

L'invention porte sur un procédé mis en oeuvre par ordinateur de détermination d'un motif d'auto-assemblage d'un copolymère à blocs confiné à l'intérieur d'un contour fermé.

L'auto-assemblage dirigé de copolymères à blocs (DSA, de l'anglais « Direct Self-Assembly ») attire une attention croissante en tant que technologie pouvant permettre la réalisation de motifs de surface à l'échelle nanométrique, en surmontant les limites de résolution de la lithographie. En particulier, cette technique semble bien adaptée à la réalisation de motifs de lignes (pistes conductrices) et trous (VIAs) dans les circuits intégrés de la prochaine génération.

La séparation des phases des copolymères à blocs (BCP, de l'anglais « Block Co-Polymers ») forme, par auto-assemblage, des nano-domaines en forme de cylindres, sphères ou lamelles dont l'échelle spatiale varie de quelques nanomètres à plusieurs dizaines de nanomètres. Parmi ces différentes structures, les domaines cylindriques s'avèrent particulièrement adaptés à la réalisation de trous d'interconnexion dans les circuits intégrés. Selon une approche connue sous le nom de graphoépitaxie, l'auto-assemblage d'un BCP se fait à l'intérieur d'un contour de guidage préalablement réalisé sur une surface. Le fort confinement latéral induit par les parois de ce contour de guidage modifie de manière prévisible l'agencement « naturel » en surface libre des nano-domaines (un motif hexagonal dans le cas de domaines cylindriques perpendiculaires au substrat). Ainsi, il a été prouvé que l'utilisation d'un contour de guidage approprié permet la réalisation d'un agencement arbitraire de nano-cylindres, qui peut correspondre à un motif de trous d'interconnexion dans un circuit intégré.

Les contours de guidage pour graphoépitaxie sont typiquement réalisés par lithographie, et présentent une forme qui présente inévitablement des écarts par rapport à celle désirée et définie par le masque de lithographie. Il est donc nécessaire de vérifier que le motif d'auto-assemblage dirigé obtenu à partir d'un contour de guidage « réel » - visualisé, par exemple, par microscopie électronique à balayage - sera suffisamment proche du motif attendu, en fonction de l'application visée. Pour cela, on peut avoir recours à des simulations numériques basées sur des modèles physiques du processus d'auto-assemblage. Ces modèles physiques peuvent être séparés en deux grandes familles : les modèles particulaires et ceux basés sur des champs d'énergie. On peut citer, à titre d'exemples non limitatifs, les publications suivantes :

- Pour les modèles particulaires:

    - « Dissipative particle dynamics study on directed self-assembly in holes » T. Nakano ; M. Matsukuma ; K. Matsuzaki ; M. Muramatsu ; T. Tomita ; T. Kitano Proc. SPIE 8680, Alternative Lithographie Technologies V, 86801J (March 26, 2013)
    - « Molecular Dynamics Study of the Role of the Free Surface on Block Copolymer Thin Film Morphology and Alignment Christopher Forrey » , Kevin G. Yager , and Samuel P. Broadaway ACS Nano, 2011, 5 (4), pp 2895-2907

- Pour les modèles basés sur des champs d'énergie :

    - « Computational simulation of block copolymer directed self-assembly in small topographical guiding templates » He Yi ; Azat Latypov ; H.-S. Philip Wong Proc. SPIE 8680, Alternative Lithographie Technologies V, 86801L (March 26, 2013)
    - « Large-scale dynamics of directed self-assembly defects on chemically pre-patterned surface » Kenji Yoshimoto ; Takashi Taniguchi Proc. SPIE 8680, Alternative Lithographic Technologies V, 86801I (March 26, 2013);

Toutes ces méthodes mettent en oeuvre des algorithmes itératifs ; ils sont donc beaucoup trop lents pour être utilisés en phase de production. Par ailleurs, il est connu de l'art antérieur (« Directed self-assembly of a colloidal kagome lattice », Chen et al., Nature, volume 469, pages 381-384 (20 January 2011)) d'observer un procédé d'auto-assemblage dirigé de sphères colloïdales formant des réseaux kagomé colloïdaux à partir de sphères de taille micrométrique à l'aide de la microscopie à fluorescence. Cependant, cette méthode ne concerne pas les copolymères à blocs, ne cherche pas à définir un contour de guidage ou de choisir un contour de référence à partir de modèles.

L'invention vise à remédier à cet inconvénient de l'art antérieur en procurant un procédé de détermination d'un motif d'auto-assemblage d'un copolymère à blocs confiné à l'intérieur d'un contour fermé qui soit plus rapide que les méthodes connues tout en étant suffisamment précis et prédictif. Plus particulièrement, l'invention vise à procurer un procédé suffisamment rapide pour pouvoir être utilisé pour déterminer de tels motifs d'auto-assemblage sur toute la surface d'un circuit intégré.

Conformément à l'invention, ce but est atteint à l'aide d'un procédé basé sur une transformation géométrique simple d'un contour « de référence », repéré au sein d'une base de données stockée dans la mémoire d'un ordinateur et identifié comme étant le plus proche du profil de guidage considéré.

Ainsi, un objet de l'invention permettant d'atteindre ce but est un procédé de détermination d'un motif d'auto-assemblage d'un copolymère à blocs confiné à l'intérieur d'un contour fermé, dit contour de guidage, comportant les étapes suivantes, mises en oeuvre par ordinateur :

a) choisir dans une base de données un contour fermé dit de référence approchant ledit contour de guidage, un motif d'auto-assemblage dudit copolymère à blocs dit motif de référence étant associé audit contour de référence ;
b) appliquer une transformation géométrique à une pluralité de points dudit motif de référence pour les convertir en des points respectifs, dits points images, du motif d'auto-assemblage à déterminer, ladite transformation géométrique étant fonction d'une transformation géométrique permettant de passer dudit contour de référence audit contour de guidage, lesdits points images définissant ledit motif d'auto-assemblage.

Selon différents modes de réalisation particuliers d'un tel procédé :

- Ledit motif de référence peut comprendre au moins une première phase et une deuxième phase, lesdits points dudit motif de référence étant choisis de manière à échantillonner une frontière entre ladite première phase et ladite deuxième phase.
- Ladite base de données peut contenir une pluralité de contours fermés échantillonnés selon un procédé d'échantillonnage; le procédé comprenant une étape préliminaire d'échantillonnage dudit contour de guidage selon le même procédé d'échantillonnage et avec un même nombre de points d'échantillonnage qu'au moins certains des contours fermés contenus dans ladite base de données.
- La distance curviligne entre deux dits points d'échantillonnage peut être, pour tous les contours fermés contenus dans ladite base de données et pour ledit contour de guidage, comprise entre la moitié et le double d'une longueur prédéfinie $s_0$.
- Ladite longueur prédéfinie $s_0$ peut être telle que $L_0/5 \leq s_0 \leq 5L_0$, de préférence $L_0/2 \leq s_0 \leq 2L_0$ et de manière encore plus préférée $L_0/2 \leq s_0 \leq L_0$, où $L_0$ est une période naturelle dudit copolymère à blocs.
- Ladite étape a) peut comprendre :

a1) la sélection des contours fermés de ladite base comportant autant de points d'échantillonnage que ledit contour de guidage ; et
a2) le choix, parmi les contours fermés ainsi sélectionnés, de celui qui minimise un critère de distance fonction des coordonnées des points d'échantillonnage dudit contour fermé et dudit contour de guidage.

- Ledit critère de distance peut être une distance quadratique entre les coefficients complexes obtenus par transformée de Fourier discrète de deux vecteurs de nombres complexes représentant, respectivement, les coordonnées des points d'échantillonnage dudit contour fermé et dudit contour de guidage.
- Ladite base de données peut contenir, pour chaque dit contour fermé, un ensemble de données définissant une triangulation d'une surface délimitée par ledit contour, et ladite étape b) peut comprendre :

b1) la détermination d'une triangulation d'une surface délimitée par ledit contour de guidage, chaque triangle et chaque sommet de ladite triangulation étant associés à un triangle et à un sommet respectifs de ladite triangulation de la surface délimitée par le contour de référence ; et
b2) pour une pluralité de points dudit motif de référence, dont chacun est contenu dans un triangle de ladite triangulation de la surface délimitée par contour de référence, la détermination d'un point image contenu dans le triangle associé de ladite triangulation de la surface délimitée par ledit contour de guidage.

- Chaque dit point dudit motif de référence et son point image peuvent présenter, par rapport aux triangles dans lesquels ils sont respectivement contenus, les mêmes coordonnées barycentriques.
- Ladite étape b2) peut comprendre également :

b2') pour chacun ou au moins un desdits points dudit motif de référence, la construction d'au moins un triangle additionnel contenant ledit point et dont les sommets sont situés sur ledit contour de référence ;
le point image dudit ou de chaque dit point du motif de référence présentant, par rapport au triangle dont il est contenu, des coordonnées barycentriques obtenues par combinaison linéaire des coordonnées barycentriques dudit point du motif de référence par rapport aux triangles dans lesquels il est contenu.

- Les triangulations desdits contours peuvent être effectuées en prenant, en tant que sommets, au moins une partie desdits points d'échantillonnages.

- Un tel procédé peut comporter également l'étape suivante :
  c) utiliser un algorithme de modélisation physique d'auto-assemblage dudit copolymère à blocs (autrement dit, un algorithme de modélisation physique) pour affiner ledit motif d'auto-assemblage définit par les points image, en prenant lesdits points image en tant que données d'initialisation.

Un autre objet de l'invention est un produit programme d'ordinateur comprenant des instructions de code de programme pour l'exécution des étapes d'un tel procédé lorsque ledit programme est exécuté sur un ordinateur.

D'autres caractéristiques, détails et avantages de l'invention ressortiront à la lecture de la description faite en référence aux dessins annexés donnés à titre d'exemple dans lesquels :

- La figure 1A est l'image en microscopie électronique à balayage d'un contour de guidage et du motif d'auto-assemblage associé ;
- La figure 1B montre le même contour échantillonné avec sa triangulation ;
- Les figures 2A - 2G représentent des profils de référence respectifs pouvant être stockés dans une base de données ;
- La figure 3 illustrent l'utilisation de triangles additionnels dans un procédé selon un mode de réalisation particulier de l'invention ; et
- La figure 4 illustre le résultat technique de l'invention.

L'invention sera décrite à l'aide d'exemples dans lesquels le copolymère à blocs considéré est du type PS-b-PMMA cylindrique (c'est-à-dire formant par auto-assemblage des nano-cylindres). Cependant l'invention peut s'appliquer à d'autres types de copolymères à blocs (di-blocs, tri-blocs en étoile ou de structure linéaire...) et à des mélanges de tels copolymères. Il n'y a pas non plus de restriction sur le type de procédé de graphoépitaxie utilisé (« solvent annealing », c'est-à-dire « recuit en vapeur de solvant » ; « grafting layer », c'est-à-dire « couche de greffage » etc...).

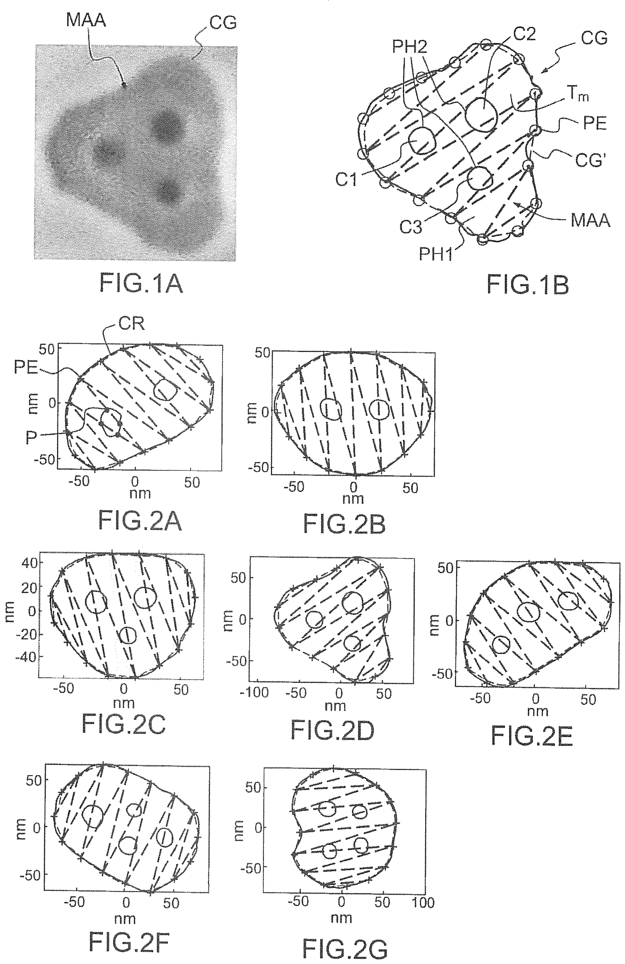

La figure 1A illustre une image de microscopie électronique à balayage d'un motif d'auto-assemblage MAA d'un copolymère à blocs (PS-b-PMMA) confiné par un contour fermé de guidage CG de forme trilobée. Ce motif comprend une première phase continue (référence PH1 sur la figure 1B) et une seconde phase discrète (référence PH2 sur la figure 1B) formée par trois cylindres (C1, C2 et C3 sur la figure 1B). L'image est numérisée et acquise par un ordinateur. Ensuite, comme le montre la figure 1B, le contour CG est échantillonné. Dans le cas présent, l'échantillonnage est régulier, c'est-à-dire avec des points d'échantillonnage PE agencés sensiblement à équidistance les uns des autres le long du contour. Pour des raisons d'efficacité de calcul qui seront expliquées par la suite, il est préférable que le nombre de points d'échantillonnage soit une puissance de 2 - ici $16=2^4$. Les points sont ordonnés en parcourant le contour dans le sens trigonométrique. La référence CG' correspond au polygone, dont les sommets coïncident avec les points d'échantillonnage, qui approche le contour de guidage (contour discrétisé). Les frontières des domaines cylindriques C1, C2 et C3 sont également échantillonnées, bien que cela ne soit pas représenté sur la figure.

Ensuite, une triangulation de la surface délimitée par le contour CG (ou, plus exactement, par le contour discrétisé CG') est définie en prenant les points d'échantillonnage E comme sommets des triangles. La référence $T_m$ sur la figure 1B identifie l'un de ces triangles.

Une pluralité d'images discrétisées du type de la figure 1B, correspondant à des contours différents guidant l'auto-assemblage d'un même copolymère à blocs, sont stockées dans la mémoire de l'ordinateur pour former une base de données, ou librairie. Ces images peuvent correspondre à des échantillons réels ou être obtenues à partir d'algorithmes de modélisation physiques de grande précision. Les figures 2A à 2G montrent les images d'une telle base de données.

Dans la base de données, chaque image peut être représentée par :

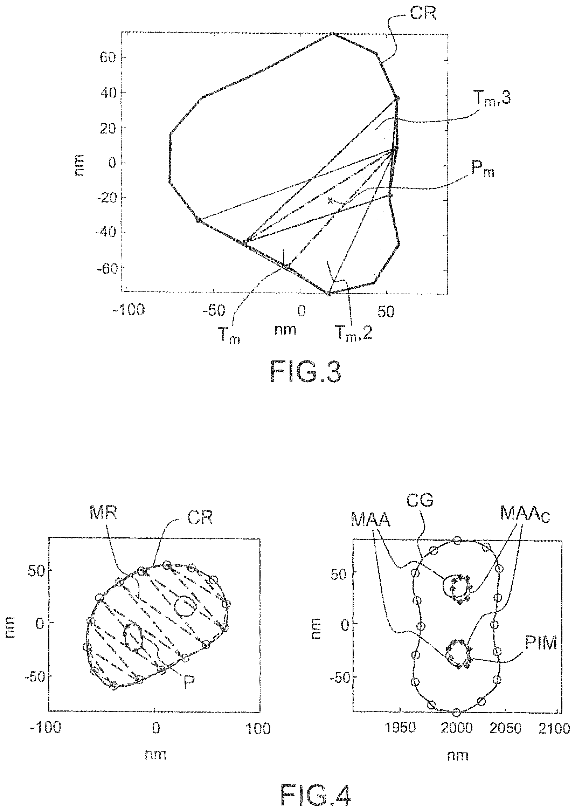

- Les coordonnées des points d'échantillonnage PE du contour de guidage ; en particulier, chaque point d'échantillonnage peut être représenté par un nombre complexe $z=x+jy$, où « j » est l'unité imaginaire et $(x, y)$ les coordonnées cartésiennes du point d'échantillonnage.
- Des données identifiant la triangulation ; par exemple, chaque triangle peut être identifié par trois nombres entiers identifiant les points d'échantillonnage lui servant de sommets.
- Les coordonnées des points d'échantillonnage P (voir la figure 2A) des frontières des domaines cylindriques (plus généralement, des frontières entre phases distinctes du motif d'auto-assemblage). Plus particulièrement, chacun de ces points peut être identifié par ses coordonnées barycentriques dans le triangle le contenant, ainsi que par un identifiant de ce triangle. Comme cela sera expliqué plus loin, à l'aide de la figure 3, un même point P peut se trouver à l'intérieur de plusieurs triangles, auquel cas plusieurs jeux de coordonnées barycentriques peuvent être stockés dans la base de données. On rappelle que les coordonnées barycentriques d'un point par rapport à un triangle sont les poids (éventuellement négatifs, si le point en question est à l'extérieur du triangle) qu'il faut donner aux sommets dudit triangle pour que ledit point en soit le barycentre.
- La transformée de Fourier du contour échantillonné peut également être stockée dans la base de données, ce qui évite d'avoir à la calculer ultérieurement. Comme expliqué plus haut, chaque point d'échantillonnage peut être identifié par un nombre complexe ; ainsi le contour discrétisé CG' est représenté par un vecteur complexe, dont la transformée de Fourier peut être calculée. La raison pour laquelle on choisit de préférence un nombre de points d'échantillonnage égal à une puissance de deux est que cela permet d'utiliser l'algorithme de transformée de Fourier rapide (FFT).

Avantageusement, la distance curviligne entre deux points d'échantillonnage est approximativement la même pour toutes les images de la base de données, et plus précisément est comprise entre la moitié et le double d'une longueur de référence $s_0$. Plus particulièrement, on peut imposer que la distance « s » entre une paire arbitraire de points d'échantillonnage adjacents satisfasse l'inégalité $|s-s_0| \leq 0,4 \cdot s_0$. La longueur de référence est de préférence du même ordre de grandeur de la période naturelle $L_0$ du copolymère, c'est-à-dire de la distance entre domaines (par exemple cylindriques) en cas d'auto-assemblage libre, non contraint par un contour de guidage, du copolymère. Par exemple on peut avoir $L_0/5 \leq s_0 \leq 5L_0$, plus particulièrement $L_0/2 \leq s_0 \leq 2L_0$ et plus particulièrement encore $L_0/2 \leq s_0 \leq L_0$, et à titre d'exemple particulier $s_0 = L_0$.

Le nombre de points d'échantillonnage d'un contour de longueur L peut alors être donné par :

$$N = 2^{E\left(0,5 + \frac{\log\left(\frac{L}{s_0}\right)}{\log(2)}\right)}$$

où « E » est la fonction « partie entière ».

Pour la mise en oeuvre d'un procédé selon l'invention, il est préférable, pour des raisons qui deviendront apparentes par la suite, que la numérotation des points d'échantillonnage ne soit pas arbitraire.

Pour cela, un contour de guidage particulier est préalablement centré de façon à ce que son centre de gravité « g » soit placé à l'origine d'un repère cartésien. Ensuite, le contour est échantillonné dans le sens trigonométrique. Comme cela a été expliqué plus haut, l'ensemble des points d'échantillonnage résultant est alors constitué de nombres complexes $(c_i)_{1 \leq i \leq N}$ tels que la distance curvilinéaire les séparant soit égale à $s = \dfrac{L}{N}$ et tels que l'axe des abscisses et le vecteur $\overrightarrow{gc_1}$ forment entre eux un angle égal à $\theta_0 = \dfrac{\left(Arg(\widetilde{c}_2) + Arg(\widetilde{c}_N)\right)}{2}$, où $(\widetilde{c}_i)_{1 \leq i \leq N}$ sont les valeurs de la transformée de Fourier discrète de $(c_i)_{1 \leq i \leq N}$ et « Arg » est la fonction donnant l'argument d'un nombre complexe. Il convient de noter que $\theta_0$ est l'orientation du grand axe de l'ellipse approchant le contour.

La triangulation de la surface délimitée par le contour de guidage ainsi échantillonné peut alors être effectuée de la manière suivante :

- Le premier triangle $T_1$ a pour sommets les points $p_1 = c_1$, $q_1 = c_2$ et $r_1 = c_N$.

- Les triangles $\left(T_{2i}\right)_{1 \leq i \leq \frac{N}{2}-2}$ ont pour sommets les points $p_{2i} = c_{i+1}$, $q_{2i} = c_{i+2}$ et $r_{2i} = c_{N-i+1}$.

- Les triangles $\left(T_{2i+1}\right)_{1 \leq i \leq \frac{N}{2}-2}$ ont pour sommets les points $p_{2i+1} = c_{i+2}$, $q_{2i+1} = c_{N-i}$ et $r_{2i+1} = c_{N-i+1}$.

Il peut être intéressant de noter que cette triangulation n'est pas, en général, une triangulation de Delaunay, sauf si le polygone CG' est convexe. Si la triangulation n'est pas de Delaunay, il se peut que des triangles se superposent, et que donc un point particulier du motif MAA soit contenu dans plusieurs triangles. Cela n'a pas d'importance pour la mise en oeuvre du procédé de l'invention.

Une fois la base de données constituée, on se pose le problème de déterminer le motif d'auto-assemblage du même copolymère à l'intérieur d'un contour de guidage autre que ceux contenus dans la base. Pour ce faire, ce contour (sans le copolymère à l'intérieur) doit être échantillonné comme expliqué plus haut ; le nombre N de points d'échantillonnage du contour doit être le même que pour au moins une partie de la base de données. Une triangulation de la surface délimitée par ce contour doit en outre être déterminée de la même manière que lors de la construction de ladite base de données.

Ensuite, il est nécessaire de déterminer, parmi les contours stockés dans la base de données avec N points d'échantillonnage, celui qui s'approche le plus du contour de guidage pour lequel on veut déterminer le motif d'auto-assemblage ; le contour ainsi déterminé sera appelé « contour de référence » dans la suite. Pour ce faire, il faut définir un critère de similitude entre contours. Avantageusement, mais pas de manière limitative, il pourra s'agir de la distance quadratique entre les coefficients de Fourier des contours :

$$\sum_{i=1}^{N} \left( \left| \tilde{c}'_i \right|^2 - \left| \tilde{c}_i \right|^2 \right)$$

où $\tilde{c}_i$ est le i-ème coefficient de Fourier du contour de guidage à caractériser et $\tilde{c}'_i$ celui du contour de la base de données auquel on le compare. La transformée de Fourier discrète d'un contour est calculée comme cela a été expliqué plus haut, en représentant chaque point d'échantillonnage par un nombre complexe. La numérotation des points d'échantillonnage doit suivre un critère commun à tous les contours (par exemple, mais pas limitativement, celui expliqué plus haut) afin de rendre significative la distance quadratique ainsi calculée. Il convient de noter que les informations sur la rotation du contour et sur l'origine de l'échantillonnage sont contenues dans la phase du spectre de Fourier. Elles ne sont donc pas prises en compte lors de la comparaison des contours, ce qui est souhaitable.

En d'autres termes, on applique à la base de données un double filtrage : d'abord on présélectionne les contours qui présentent une longueur proche à celle du contour à caractériser (par comptage du nombre de points d'échantillonnage), puis on sélectionne un seul contour de référence en appliquant un critère de similitude approprié.

Une fois le contour de référence trouvé dans la librairie, on tourne le guide à caractériser de façon à minimiser la distance entre les deux jeux de points d'échantillonnage. Cette position peut être obtenue avec une bonne approximation en appliquant une rotation d'angle égal à celui formé par les vecteurs $\overrightarrow{gc_1}$ et $\overrightarrow{g'c'_1}$, g' étant le centre de gravité du contour de référence.

A ce point, on peut procéder à la transformation géométrique qui convertit le motif d'auto-assemblage associé au contour de référence (« motif de référence ») - connu et stocké dans la base de données - en un motif approchant celui qui sera obtenu en réalisant l'auto-assemblage du même copolymère à blocs à l'intérieur du contour de guidage à caractériser.

Cette transformation géométrique s'appuie sur les triangulations, et plus précisément sur la fait que chaque point d'échantillonnage du contour à caractériser est associé à (« est l'image de ») un point d'échantillonnage respectif du contour de référence, et chaque triangle de la triangulation de la surface délimitée par le contour à caractériser est associé à un triangle respectif de la triangulation de la surface délimitée par le contour de référence. Ainsi, on pourra identifier une pluralité de points du motif de référence, déterminer le triangle dans lequel chacun de ces points est contenu et lui associer un point image contenu dans le triangle associé de la triangulation de la surface délimitée par le contour à caractériser. Plus particulièrement, un point du motif de référence peut être caractérisé par ses coordonnées barycentriques ($\alpha$, $\beta$, $\gamma$) par rapport au triangle le contenant, et son point image par des coordonnées barycentriques identiques ($\alpha_{IM}$, $\beta_{IM}$, $\gamma_{IM}$)=($\alpha$, $\beta$, $\gamma$) par rapport au triangle associé.

Déterminer dans quel triangle se trouve un point déterminé du motif de référence ne pose pas de difficulté particulière. Par exemple on peut parcourir séquentiellement tous les triangles en calculant les coordonnées barycentriques du point par rapport à chaque dit triangle et s'arrêter lorsque $\alpha+\beta+\gamma=1$ avec $\alpha$, $\beta$ et $\gamma$ compris entre 0 et 1. Il est possible à ce stade de stocker cette information dans la base de données.

Dans un souci d'efficacité, il est opportun de n'appliquer cette transformation géométrique qu'aux points d'échantillonnage de la frontière des domaines cylindriques (plus généralement : de la frontière entre phases) dans le motif de référence (point « P » sur la figure 2A). En effet la connaissance de ces points suffit pour reconstituer de manière approchée un motif d'auto-assemblage.

Une difficulté vient du fait que l'échelle de longueur caractéristique de la triangulation qui sert de base à la transformation géométrique est généralement plus petite que la longueur de paroi du contour de guidage influençant le positionnement des domaines cylindriques formés par l'auto-assemblage du copolymère. Le présent inventeur s'est donc rendu compte qu'il est avantageux d'utiliser également des triangles additionnels avec des longueurs de base plus grandes (n'ayant pas deux sommets constitués par des points d'échantillonnage adjacents) lors de la transformation géométrique. En particulier, il a été trouvé qu'un nombre de triangle nbTri = 3 est généralement satisfaisant.

Les triangles additionnels associés au triangle $T_m$ de la triangulation peuvent être en nombre paire et être définis comme suit :

Si m = 2i+1 est impair (« i » étant un entier) :

Le triangle additionnel $T_{m,2k}$ a pour sommet les points $p_{m,2k} = c_{i+2-k}$, $q_{m,2k} = c_{i+2+k}$ et $r_{m,2k} = c_{N-i}$.
Le triangle additionnel $T_{m,2k+1}$ a pour sommet les points $p_{m,2k+1} = c_{i+2}$, $q_{m,2k} = c_{N-i-k}$ et $r_{m,2k} = c_{N-i+1+k}$.

Si m = 2i est pair :

Le triangle additionnel $T_{m,2k}$ a pour sommet les points $p_{m,2k} = c_{i+1-k}$, $q_{m,2k} = c_{i+2+k}$ et $r_{m,2k} = c_{N-i+1}$.
Le triangle additionnel $T_{m,2k+1}$ a pour sommet les points $p_{m,2k+1} = c_{i+1}$, $q_{m,2k} = c_{N-i+1-k}$ et $r_{m,2k} = c_{N-i+1+k}$.

avec k compris entre 1 et (nbTri - 1)/2. Dans les formules ci-dessus, il se peut que les indices des points c ne soient pas compris entre 1 et N. Dans ce cas, le triangle ne peut pas être construit et on utilisera un jeu de coordonnées barycentriques restreint.

La figure 3 montre un point $P_m$ situé à l'intérieur d'un triangle $T_m$ faisant partie de la triangulation du contour de référence CR, ainsi que de deux triangles additionnels $T_{m,2}$ et $T_{m,3}$. Dans ce cas, nbTri=3.

Si on utilise des triangles additionnels, la transformation géométrique doit être redéfinie. Si on considère un point P du motif de référence MR contenu dans nbTri triangles (un triangle $T_m$ appartenant à la triangulation et nbTri-1 triangles additionnels), le point image se trouvera dans le triangle de la triangulation du contour à caractériser associé à $T_m$ et ses coordonnées barycentriques par rapport à ce triangle seront données par une moyenne pondérées des coordonnées barycentriques dans les différents triangles du contour de référence. Les coefficients de pondération seront typiquement choisis de telle sorte que l'image d'un point situé sur le contour de référence se trouve sur le contour à caractériser.

Un point P quelconque du guide se situant dans le triangle $T_{m,1}$ a pour image le point PIM tel que :

$$P_{PIM} = \frac{\sum_{k=1}^{nbTri} \prod_{l \neq k} \alpha_l \beta_l \gamma_l (\alpha_k p_k + \beta_k q_k + \gamma_k r_k)}{\sum_{k=1}^{nbTri} \prod_{l \neq k} \alpha_l \beta_l \gamma_l}$$

où :

- $P_{PIM}$ est le nombre complexe représentant les coordonnées cartésiennes du point image PIM ;
- L'indice « k » identifie les nbTri triangles $T_k$ contenant le point P - typiquement un triangle appartenant à la triangulation de la surface délimitée par le contour de référence et (nbTri-1) triangles additionnels ;
- $p_k$, $q_k$ et $r_k$ sont les nombres complexes représentant les coordonnées cartésiennes des sommets du triangle $T_k$ ;
- $\alpha_k$, $\beta_k$, $\gamma_k$ et $\alpha_l$, $\beta_l$, $\gamma_l$ sont respectivement les coordonnées barycentriques du point P dans les triangles $T_k$ et $T_l$.

Dans le cas nbTri=1 (ce qui signifie qu'il n'y a pas de triangles additionnels) l'équation ci-dessus se simplifie de la manière suivante :

$$P_{PIM} = \alpha p + \beta q + \gamma r$$

La partie droite et la partie gauche de la figure 4 montrent, respectivement, un contour de guidage CG à caractériser et le contour de référence correspondant CR, avec le motif de référence MR associé. Les frontières des deux domaines cylindriques contenus dans ce motif de référence MR sont échantillonnées ; l'un des points d'échantillonnage est identifié par la référence P. Une transformation géométrique telle que décrite ci-dessus, avec nbTri=3, a été appliquée pour trouver les points images de ces points d'échantillonnage à l'intérieur du contour CG à caractériser ; par exemple, la référence PIM identifie le point image du point P précité. Sur la partie de droite de la figure, la référence MAAc identifie le motif d'auto-assemblage calculé, formé par tous les points images des points d'échantillonnage des frontières des domaines cylindriques du motif de référence. On peut vérifier que ce motif calculé est très proche du « vrai » motif d'auto-assemblage MAA, déterminé ici expérimentalement (mais on aurait pu avoir recours également à une modélisation physique). Optionnellement, il est possible d'utiliser le motif d'auto-assemblage calculé MAAc pour initialiser un algorithme de modélisation de l'auto-assemblage ; dans ce cas, on peut s'attendre à ce que ce dernier converge en quelques itérations seulement. Il peut s'agir par exemple d'un algorithme de modélisation physique.

Plusieurs variantes du procédé qui vient d'être décrit peuvent être envisagées sans sortir du cadre de la présente invention. Par exemple, d'autres critères de similitude que la distance quadratique entre les coefficients de Fourier des contours. Egalement, on peut envisager l'utilisation de deux échantillonnages différents de chaque contour, l'un destiné à la mesure de la similitude entre contours et l'autre à la triangulation. Au niveau de l'échantillonnage proprement dit, différents procédés peuvent être utilisés dans la mesure où un même procédé est utilisé pour les contours de référence et le contour à étudier. Un tel procédé d'échantillonnage doit en particulier permettre d'identifier N points sur le contour et de fixer la position du premier point sur ce contour ainsi qu'un sens de circulation sur le contour Par ailleurs, la relation entre un point du motif de référence et son point image dans le motif d'auto-assemblage calculé peut ne pas être basée sur les coordonnées barycentriques des points. Il peut s'agir, plus généralement, de toute relation issue d'une transformation géométrique fonction de la transformation géométrique permettant de passer du contour de référence au contour de guidage considéré.

## Revendications

1. Procédé de détermination d'un motif d'auto-assemblage (MAA) d'un copolymère à blocs confiné à l'intérieur d'un contour fermé, dit contour de guidage (CG), comportant les étapes suivantes, mises en oeuvre par ordinateur :

   a) choisir dans une base de données un contour fermé dit de référence (CR) approchant ledit contour de guidage, un motif d'auto-assemblage dudit copolymère à blocs dit motif de référence (MR) étant associé audit contour de référence ;
   b) appliquer une transformation géométrique à une pluralité de points (P, $P_m$) dudit motif de référence pour les convertir en des points respectifs, dits points images (PIM), du motif d'auto-assemblage à déterminer, ladite transformation géométrique étant fonction d'une transformation géométrique permettant de passer dudit contour de référence audit contour de guidage, lesdits points image définissant ledit motif d'auto-assemblage.

2. Procédé selon la revendication 1 dans lequel ledit motif de référence comprend au moins une première phase (PH1) et une deuxième phase (PH2), lesdits points dudit motif de référence étant choisis de manière à échantillonner une frontière entre ladite première phase et ladite deuxième phase.

3. Procédé selon l'une des revendications précédentes dans lequel ladite base de données contient une pluralité de contours fermés échantillonnés selon un procédé d'échantillonnage; le procédé comprenant une étape préliminaire d'échantillonnage dudit contour de guidage selon le même procédé d'échantillonnage et avec un même nombre de points d'échantillonnage (PE) qu'au moins certains des contours fermés contenus dans ladite base de données.

4. Procédé selon la revendication 3 dans lequel la distance curviligne entre deux dits points d'échantillonnage est, pour tous les contours fermés contenus dans ladite base de données et pour ledit contour de guidage, comprise entre la moitié et le double d'une longueur prédéfinie $s_0$.

5. Procédé selon la revendication 4 dans lequel ladite longueur prédéfinie $s_0$ est telle que $L_0/5 \leq s_0 \leq 5L_0$, de préférence $L_0/2 \leq s_0 \leq 2L_0$ et de manière encore plus préférée $L_0/2 \leq s_0 \leq L_0$, où $L_0$ est une période naturelle dudit copolymère à blocs.

6. Procédé selon l'une des revendications 3 à 5 dans lequel ladite étape a) comprend :

   a1) la sélection des contours fermés de ladite base comportant autant de points d'échantillonnage que ledit contour de guidage ; et
   a2) le choix, parmi les contours fermés ainsi sélectionnés, de celui qui minimise un critère de distance fonction des coordonnées des points d'échantillonnage dudit contour fermé et dudit contour de guidage.

7. Procédé selon la revendication 6 dans lequel ledit critère de distance est une distance quadratique entre les coefficients complexes obtenus par transformée de Fourier discrète de deux vecteurs de nombres complexes représentant, respectivement, les coordonnées des points d'échantillonnage dudit contour fermé et dudit contour de guidage.

8. Procédé selon l'une des revendications précédentes dans lequel ladite base de données contient, pour chaque dit contour fermé, un ensemble de données définissant une triangulation d'une surface délimitée par ledit contour, et dans lequel ladite étape b) comprend :

b1) la détermination d'une triangulation d'une surface délimitée par ledit contour de guidage, chaque triangle ($T_m$) et chaque sommet de ladite triangulation étant associés à un triangle et à un sommet respectifs de ladite triangulation de la surface délimitée par le contour de référence ; et

b2) pour une pluralité de points dudit motif de référence, dont chacun est contenu dans un triangle de ladite triangulation de la surface délimitée par contour de référence, la détermination d'un point image contenu dans le triangle associé de ladite triangulation de la surface délimitée par ledit contour de guidage.

9. Procédé selon la revendication 8 dans lequel chaque dit point dudit motif de référence et son point image présentent, par rapport aux triangles dans lesquels ils sont respectivement contenus, les mêmes coordonnées barycentriques.

10. Procédé selon la revendication 8 dans lequel ladite étape b2) comprend également :

b2') pour chacun ou au moins un desdits points dudit motif de référence, la construction d'au moins un triangle additionnel ($T_{m,2};T_{m,3}$) contenant ledit point ($P_m$) et dont les sommets sont situés sur ledit contour de référence ; le point image dudit ou de chaque dit point du motif de référence présentant, par rapport au triangle dont il est contenu, des coordonnées barycentriques obtenues par combinaison linéaire des coordonnées barycentriques dudit point du motif de référence par rapport aux triangles dans lesquels il est contenu.

11. Procédé selon l'une des revendications 8 à 10 lorsqu'elle dépend de la revendication 3 dans lequel les triangulations desdits contours sont effectuées en prenant, en tant que sommets, au moins une partie desdits points d'échantillonnages.

12. Procédé selon l'une des revendications précédentes comportant également l'étape suivante :
c) utiliserun algorithme de modélisation physique d'auto-assemblage dudit copolymère à blocs pour affiner ledit motif d'auto-assemblage défini par les points image, en prenant lesdits points image en tant que données d'initialisation.

13. Produit programme d'ordinateur comprenant des instructions de code de programme pour l'exécution des étapes d'un procédé selon l'une des revendications précédentes lorsque ledit programme est exécuté sur un ordinateur.

## Patentansprüche

1. Verfahren zum Bestimmen eines Selbstanordnungsmotivs (MAA) eines Blockcopolymers, welches im Innern einer geschlossenen Kontur eingeschlossen ist, genannt Führungskontur (CG), wobei das Verfahren folgende durch einen Computer umgesetzte Schritte beinhaltet:

a) Auswählen in einer Datenbank einer geschlossenen Kontur, genannt Referenzkontur (CR), welche sich der Führungskontur annähert, wobei ein Selbstanordnungsmotiv des Blockcopolymers, genannt Referenzmotiv (MR) mit der Referenzkontur assoziiert ist;
b) Anwenden einer geometrischen Transformation auf eine Vielzahl von Punkten (P, Pm) des Referenzmotivs, um sie in jeweilige Punkte, genannt Bildpunkte (PIM), des zu bestimmenden Selbstanordnungsmotivs umzuwandeln, wobei die geometrische Transformation von einer geometrischen Transformation abhängt, welche es ermöglicht, von der Referenzkontur zur Führungskontur zu gelangen, wobei die Bildpunkte das Selbstanordnungsmotiv definieren.

2. Verfahren nach Anspruch 1, bei welchem das Referenzmotiv mindestens eine erste Phase (PH1) und eine zweite (PH2) beinhaltet, wobei die Punkte des Referenzmotivs so gewählt werden, dass eine Grenze zwischen der ersten Phase und der zweiten Phase abgetastet wird.

3. Verfahren nach einem der vorhergehenden Ansprüche, bei welchem die Datenbank eine Vielzahl von geschlossenen Konturen beinhaltet, welche mit einem Abtastverfahren abgetastet worden sind; wobei das Verfahren einen vorbereitenden Abtastschritt der Führungskontur nach demselben Abtastverfahren und mit einer gleichen Anzahl von Abtastpunkten (PE) wie mindestens einige der in der Datenbank enthaltenen geschlossenen Konturen beinhaltet.

4. Verfahren nach Anspruch 3, bei welchem der krummlinige Abstand zwischen zwei solchen Abtastpunkten für alle in der Datenbank enthaltenen geschlossenen Konturen und für die Führungskontur zwischen der Hälfte und dem Doppelten einer vorbestimmten Länge $s_0$ beträgt.

**5.** Verfahren nach Anspruch 4, bei welchem die vorbestimmte Länge $s_0$ so gestaltet ist, dass $L_0/5s_0 \leq 2L_0$ und vorzugsweise $L_0/2s_0 < 2L_0$ und noch bevorzugterweise $L_0/5s_0 < L_0$, wobei $L_0$ eine natürliche Periode des Blockcopolymers ist.

**6.** Vorrichtung nach einem der Ansprüche 3 bis 5, bei welchem der Schritt a) Folgendes beinhaltet:

a1) Auswählen der geschlossenen Konturen aus der Datenbank, welche ebenso viele Abtastpunkte wie die Führungskontur beinhalten; und

a2) Wählen, aus den solchermaßen ausgewählten geschlossenen Konturen, derjenigen Kontur, welche ein Abstandskriterium abhängig von den Koordinaten der Abtastpunkte der geschlossenen Kontur und der Führungskontur minimiert.

**7.** Verfahren nach Anspruch 6, bei welchem das Abstandskriterium ein quadratischer Abstand zwischen den komplexen Koeffizienten ist, welche mittels diskreter FourierTransformation zweier Vektoren von komplexen Zahlen erzielt wird, welche jeweils die Koordinaten der Abtastpunkte der geschlossenen Kontur und der Führungskontur darstellen.

**8.** Verfahren nach einem der vorhergehenden Ansprüche, bei welchem die Datenbank für jede geschlossene Kontur genannte Kontur eine Gruppe von Daten beinhaltet, welche eine Triangulierung einer durch die Kontur begrenzten Fläche definiert, und bei welcher der Schritt b) Folgendes beinhaltet:

b1) Bestimmen einer Triangulation einer durch die Führungskontur begrenzten Fläche, wobei jedes Dreieck ($T_m$) und jede Spitze der Triangulation mit einem jeweiligen Dreieck und einer jeweiligen Spitze der Triangulation der durch die Referenzkontur begrenzten Fläche assoziiert sind; und

b2) für eine Vielzahl von Punkten des Referenzmotivs, von denen ein jeder in einem Dreieck der Triangulation der durch die Referenzkontur begrenzten Fläche enthalten ist, Bestimmen eines Bildpunktes, welcher in dem assoziierten Dreieck der Triangulation der durch die Führungskontur begrenzten Fläche enthalten ist.

**9.** Verfahren nach Anspruch 8, bei welchem jeder Punkt des Referenzmotivs und dessen Bildpunkt in Bezug auf die Dreiecke, in welchen sie jeweils enthalten sind, dieselben baryzentrischen Koordinaten aufweisen.

**10.** Verfahren nach Anspruch 8, bei welchem der Schritt b2) ebenfalls Folgendes beinhaltet:

b2') für jeden oder für mindestens einen Punkt des Referenzmotivs, Konstruieren von mindestens einem zusätzlichen Dreieck ($T_{m,2}$;$T_{m,3}$), welches den Punkt ($P_m$) enthält und dessen Spitzen sich auf der Referenzkontur befinden;

wobei der Bildpunkt des oder eines jeden Punktes des Referenzmotivs in Bezug auf das Dreieck, in dem es enthalten ist, baryzentrische Koordinaten aufweist, welche durch lineare Kombination der baryzentrischen Koordinaten des Punktes des Referenzmotivs in Bezug auf die Dreiecke, in denen es enthalten ist, erzielt werden.

**11.** Verfahren nach einem der Ansprüche 8 bis 10, in Abhängigkeit von Anspruch 3, bei welchem die Triangulationen der Konturen durchgeführt werden, indem man als Spitzen mindestens einen Teil der Abtastpunkte verwendet.

**12.** Verfahren nach einem der vorhergehenden Ansprüche, welches zudem folgenden Schritt beinhaltet:
c) Verwenden eines Algorithmus zur physischen Selbstanordnungs-Modellierung des Blockcopolymers zum Verfeinern des Selbstanordnungsmotivs, welches durch die Bildpunkte definiert wird, indem man die Bildpunkte als Initialisierungsdaten verwendet.

**13.** Computerprogrammprodukt, welches Programmecodeanweisungen zur Ausführung der Schritte eines Verfahrens nach einem der vorhergehenden Ansprüche beinhaltet, wenn das Programm auf einem Computer ausgeführt wird.

**Claims**

**1.** A method for determining a self-assembly pattern (MAA) of a block copolymer confined inside a closed outline called the guiding outline (CG), including the following steps, which are implemented by computer:

a) choosing in a database a closed outline called the reference outline (CR) that is similar to said guiding outline, a self-assembly pattern of said block copolymer, which is called the reference pattern (MR), being associated with said reference outline;

b) applying a geometric transformation to a plurality of points ($P$, $P_m$) of said reference pattern in order to convert them to respective points called image points (PIM) of the self-assembly pattern to be determined, said geometric transformation being a function of a geometric transformation allowing said reference outline to be converted to said guiding outline, said image points defining said self-assembly pattern.

**2.** The method according to claim 1, wherein said reference pattern comprises at least one first phase (PH1) and one second phase (PH2), said points of said reference pattern being chosen so as to sample a boundary between said first phase and said second phase.

**3.** The method according to one of the preceding claims, wherein said database contains a plurality of closed outlines sampled according to a sampling method; the method comprising a prior step of sampling said guiding outline according to the same sampling method and with the same number of sampling points (PE) as at least some of the closed outlines contained in said database.

**4.** The method according to claim 3, wherein the curvilinear distance between two said sampling points is, for all the closed outlines contained in said database and for said guiding outline, comprised between half and twice a predefined length $s_0$.

**5.** The method according to claim 4, wherein said predefined length $s_0$ is such that $L_0/5 \leq s_0 \leq 5L_0$, preferably $L_0/2 \leq s_0 \leq 2L_0$ and even more preferably $L_0/2 \leq s_0 \leq L_0$, where $L_0$ is a natural period of said block copolymer.

**6.** The method according to one of claims 3 to 5, wherein said step a) comprises:

a1) selecting closed outlines from said database including as many sampling points as said guiding outline; and
a2) choosing, from the closed outlines thus selected, that which minimizes a distance criterion dependent on the coordinates of the sampling points of said closed outline and of said guiding outline.

**7.** The method according to claim 6, wherein said distance criterion is a quadratic distance between the complex coefficients obtained by discrete Fourier transform of two vectors of complex numbers representing the coordinates of the sampling points of said closed outline and of said guiding outline, respectively.

**8.** The method according to one of the preceding claims, wherein said database contains, for each said closed outline, a dataset defining a triangulation of an area bounded by said outline, and wherein said step b) comprises:

b1) determining a triangulation of an area bounded by said guiding outline, each triangle ($T_m$) and each apex of said triangulation being associated with a respective triangle and a respective apex of said triangulation of the area bounded by the reference outline; and
b2) for a plurality of points of said reference pattern, each of which is contained in a triangle of said triangulation of the area bounded by the reference outline, determining an image point contained in the associated triangle of said triangulation of the area bounded by said guiding outline.

**9.** The method according to claim 8, wherein each said point of said reference pattern and its image point have, with respect to the triangles in which they are respectively contained, the same barycentric coordinates.

**10.** The method according to claim 8, wherein said step b2) also comprises:

b2') for each or at least one of said points of said reference pattern, constructing at least one additional triangle ($T_{m,2}$;$T_{m,3}$) containing said point ($P_m$) and the apexes of which are located on said reference outline;
the image point of said or each said point of the reference pattern having, with respect to the triangle in which it is contained, barycentric coordinates obtained by linear combination of the barycentric coordinates of said point of the reference pattern with respect to the triangles in which it is contained.

**11.** The method according to one of claims 8 to 10 when it is dependent on claim 3, wherein the triangulations of said outlines are performed while taking, by way of apexes, at least some of said sampling points.

**12.** The method according to one of the preceding claims also including the following step:
c) using a self-assembly physical modeling algorithm of said block copolymer to refine said self-assembly pattern defined by the image points, while using said image points by way of initialization data.

**13.** A computer program product comprising programming code instructions for executing the steps of a method according to one of the preceding claims when said program is executed on a computer.

FIG.1A

FIG.1B

FIG.2A

FIG.2B

FIG.2C

FIG.2D

FIG.2E

FIG.2F

FIG.2G

FIG.3

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **T. NAKANO ; M. MATSUKUMA ; K. MATSUZAKI ; M. MURAMATSU ; T. TOMITA ; T. KITANO.** Dissipative particle dynamics study on directed self-assembly in holes. *Proc. SPIE 8680, Alternative Lithographie Technologies V, 86801J,* 26 Mars 2013
- **KEVIN G. YAGER ; SAMUEL P.** Molecular Dynamics Study of the Role of the Free Surface on Block Copolymer Thin Film Morphology and Alignment Christopher Forrey. *Broadaway ACS Nano,* 2011, vol. 5 (4), 2895-2907
- **HE YI ; AZAT LATYPOV ; H.-S. PHILIP WONG.** Computational simulation of block copolymer directed self-assembly in small topographical guiding templates. *Proc. SPIE 8680, Alternative Lithographie Technologies V, 86801L,* 26 Mars 2013
- **KENJI YOSHIMOTO ; TAKASHI TANIGUCHI.** Large-scale dynamics of directed self-assembly defects on chemically pre-patterned surface. *Proc. SPIE 8680, Alternative Lithographic Technologies V, 86801I,* 26 Mars 2013
- **CHEN et al.** Directed self-assembly of a colloidal kagome lattice. *Nature,* 20 Janvier 2011, vol. 469, 381-384